# EUROPEAN PATENT SPECIFICATION

(11) **EP 4 572 607 B1**
(45) Date of publication and mention of the grant of the patent: **30.09.2026**
(21) Application number: 23841218.3
(22) Date of filing: 27.12.2023
(51) Int. Cl.: A01N 1/125

(54) **CRYOPRESERVATION FORMULATION COMPRISING COLLAGEN HYDROLYSATE**
FORMULIERUNG ZUR KRYOKONSERVIERUNG MIT KOLLAGENHYDROLYSAT
FORMULATION DE CRYOCONSERVATION COMPRENANT UN HYDROLYSAT DE COLLAGÈNE

(30) Priority: 28.12.2022 BE 202206096
(43) Date of publication of application: 25.06.2025
(73) Proprietor: Rousselot BV, 9000 Gent (BE)
(72) Inventor: OLIJVE, Joseph Hubertus, 9000 GENT (BE); LEIJTEN, Jeroen Christianus Hermanus, 9000 GENT (BE); ALLIJN, Iris Eva, 9000 GENT (BE); FERREIRA DA SILVA, Catarina Isabel, 9000 GENT (BE)
(74) Representative: EP&C
(86) International application number: PCT/EP2023/087857
(87) International publication number: WO 2024/141561

(56) References cited:
- WO-A1-2007/090504
- CN-A- 110 801 436
- JP-A- H07 255 469
- KIM BYEONGSOO ET AL: "Effects of Pressure-shift Freezing on the Structural and Physical Properties of Gelatin Hydrogel Matrices", HAN-GUG CHUGSAN SIGPUM HAG-HOEJI - KOREAN SOCIETY FOR FOOD SCIENCE OF ANIMAL RESOURCES, vol. 34, no. 1, 28 February 2014 (2014-02-28), KR, pages 33 - 39, XP093058128, ISSN: 1225-8563, Retrieved from the Internet <URL:http://koreascience.or.kr/article/JAKO201409739049115.pdf> DOI: 10.5851/kosfa.2014.34.1.33

## Description

### TECHNICAL FIELD

The current invention relates to the cryopreservation of biological materials, including cells and tissues, and cryoprotectants for use therein.

### BACKGROUND OF THE INVENTION

### Cryopreservation

Cryopreservation is a process wherein biological materials - most commonly single cell suspensions but also cellular tissues and the like - are preserved by freezing. By bringing the biological material to a frozen state, cellular enzymatic and chemical activity is brought to a stop. At any moment desired, the biological material can be thawed and the cellular activity can be resumed. Cryopreservation renders an indefinite longevity to biological materials, while in a frozen state the biological materials can also be easily transported among different (distant) laboratories. The cryopreservation of cells in particular has opened various new possibilities for medical therapies, wherein cells can be massively expanded and preserved until their use is required. The relative ease and flexibility of cryopreservation makes it an indispensable activity of cell culture labs, whether it is for the purpose of research or for therapeutic applications.

### Cell stress and toxicity in cryopreservation

Cryopreservation is most commonly at freezing temperatures such as in the order of -80 °C or less. An important limitation of cryopreservation is the inherent toxicity that the freezing process has on cells, e.g. leading to loss of cells, cellular stress, and/or changes in cellular responses. The cell toxicity in cryopreservation is related for a large part to intracellular ice formation and an osmotic imbalance in cells (Bissoyi et al. Biopreserv Biobank. 2014 Feb;12(1):23-34). With this in mind, the success in recovering cells after cryopreservation can be at least partially controlled by how the cryopreservation is managed. A preferred method of cryopreserving cells is by so-called "slow freezing". Slow freezing encompasses the cooling of cells at a controlled rate until the desired freezing temperature is reached. For example, a typical cooling rate of about 1 °C/minute is considered appropriate for many mammalian cells. The principle behind slow freezing is that it impacts the external solute concentration and thereby the cell dehydrates to certain extent and the formation of intracellular ice crystals is reduced.

### Cryoprotectants (CPAs)

Slow freezing is generally performed in conjunction with one or more cryoprotectants (i.e. cryoprotective agent, CPA). CPAs typically operate by increasing the solute concentration in cells, thereby helping to reduce freezing damage. Sulfoxides, glycols and sugars form the most common CPAs, and are typically classified as being either a permeating or a non-permeating CPA. Permeating CPAs include dimethylsulfoxide (DMSO), ethylene glycol, propylene glycol, and glycerol.

For example, JPH07255469A discloses a cryopreservation formulation based on DMSO intended for preserving rat liver parenchymal cells, the formulation comprising up to 20 wt.% hydrolysed gelatin, 10% (v/v) DMSO, 2.5 wt% serum albumin, 2.5 wt.% keratin and fetal bovine serum.

Permeating CPAs can penetrate cell membranes. In the cell, the structures' ability to hydrogen bond with water helps reduce mechanical and osmotic damage. Non-permeating CPAs typically are larger molecules and exert their protective effect outside of the cells. Non-permeating CPAs include polyethylene glycol, polyvinylpyrrolidone, methylcellulose, and sugars (e.g. sucrose, dextrose, and trehalose) (Whaley et al. Cryopreservation: An Overview of Principles and Cell-Specific Considerations. Cell Transplantation. January 2021).

### Undesirable features of CPAs

CPAs also have well-known undesirable features. These effects have been best characterized for dimethylsulfoxide (DMSO), which is also considered the most conventional CPA (e.g. also referred to as the "gold standard CPA"), although these unwanted features can often be seen for all CPAs. Some of the most relevant undesirable features are summarized as follows:
1) CPAs are toxic to cells, which seems mostly related to the apoptosis due to plasma membrane pore formation (Notman et al. J.Am. Chem. Soc. 128, 13982-13983).
2) CPAs interfere with various cellular processes and therefor may alter the cell functionality after cryopreservation. For example, changes have been reported in gene expression, cell proliferation and differentiation after the use of CPAs (Tunger et al. Scientific Reports volume 8, Article number: 14828, 2018).
3) CPAs may hamper the therapeutic potential of cells after freezing. For example, it has been found that stem cells cryopreserved with CPAs show a reduced engraftment after transplantation in patients (Mitrus et al. Randomized Controlled Trial Bone Marrow Transplant. 2018 Mar;53(3):274-280).
4) CPAs may modulate the inflammatory response in cells. The altered inflammatory response may lead to unreliable test results and/or hamper their therapeutic potential (Li et al. Front Immunol. 2021 Nov 29;12:765667).
5) The use of CPAs increases the labor intensity. Washing steps ideally ensure that all CPAs are removed from cell suspensions. This increased the required resources in the Cell Culturing labs.
6) Despite extensive washing steps, CPAs such as DMSO appear to persist in cell suspensions. DMSO contaminants cause direct side-effects on a recipient in a clinical setting (e.g. causing high blood pressure, nausea and vomiting). This limits the use in cellular and tissue transplantation, and Advanced therapy medicinal products (ATMPs).

It is typically considered as a rule of thumb, that the cell survival should be better than 80% (i.e. cell death lower than 20%). In particular for ATMPs, it is furthermore desirable that the cryopreservation formulation is GMP-grade and preferably DMSO- and/or serum-free. The cryopreservation formulations currently available, do not meet this criteria, for instance as they often require serum and/or DMSO.

### CPAs are unsuitable for several cell types

Another important limitation of conventional CPAs (e.g. DMSO, glycols, sugars) is that they are not compatible with all cell types. Among scientist world wide, it is agreed that there is no general consensus on how to predict if a specific cell type/cell line is easy or difficult to freeze. It might be stated that cell types can show the following behaviour after cryopreservation:
1 . The cells are frozen well and there is limited cell death after freezing thawing (below 20%) while cells keep their functionality;
2. The cells are frozen well, and there is limited cell death after freezing thawing (below 20%), but the cells have lost their specific functionality (quality);
3. The cells are frozen but after thawing there is more than 20% cell death and/or loss of their specific functionality (quality).

For the simplicity, the cells under point 1 are called 'easy-to-freeze' and the cells under point 2 and 3 are called respectively 'hard-to-freeze' or 'non-freezable" cells.

In order to show the discrepancy between the maturity of the cells and their behaviour in cryopreservation, some examples are given.

On the one hand, some differentiated and/or more mature cells like muscle cells and immune cells, are quite easy to freeze. While other types of differentiated cells like neural cells are difficult to freeze since these cells loose their functionality after freezing. On the other hand, some pluripotent and less differentiated and less mature cell phenotypes (e.g. fibroblasts, progenitor cells, mesenchymal stem cells, highly proliferative cells,) are easy to freeze, while embryotic stem cells (ESC's) are more difficult to freeze (Li et al. Front. Cell Dev. Biol., 14 December 2021).

Because of the absence of a clear functional description of cells in the current state of the art, the only way to describe these cells in their behaviour in cryopreservation is to refer to either "easy-to-freeze', "hard-to-freeze" or "non-freezable" cells. For example, (human) neuronal cells are typically classified "non-freezable" with CPAs, meaning that the freezing of neuronal cells leads to extremely poor recovery of cells. In addition, the recovered cells are affected in their cellular functions. Based on the aforementioned, isolated neuronal cells therefore have to be employed in experiments immediately upon isolation, which is not practical. Immortalized neuronal cell lines have also been made available for research purposes (Tremblay et al. J. Neurosci. Methods, 186, 2010, pp. 60-67), however, these cell lines do not show the same behaviour as primary neuronal cells. Neuronal cell lines are therefore deemed inappropriate for investigating neuronal-cell specific functions such as synapse formation, activity, and plasticity (Ishizuka et al. Journal of Neuroscience Methods. Volume 333, 1 March 2020).

Other examples of difficult to freeze cells include many primary (isolated cells) and/or cardiomyocytes, skeletal muscle cells, terminally-differentiated hepatocytes, terminally-differentiated fibroblasts, monocytes, dendritic cells, beta-cells, cardiomyocytes, pancreatic islets, primary human monocytes, osteocyte, and adipocytes. These cell types can be harvested form the body only in limited amounts, and remain scarce in number as they do not (or hardly) proliferate. Hence, further loss of these cells during cryopreservation is highly undesirable.

### Reducing toxicity of CPAs

In practice, proteins are commonly used to reduce the toxicity of CPAs. Human or other animal derived serum or serum proteins are examples of favoured protein sources. They may serve as universal growth supplement for supporting cell growth and differentiation. Within the field of cryopreservation, serum or serum proteins are thought to reduce toxicity by CPAs by stabilizing and protecting the cell membrane against shear stresses. However, even the use of high amounts of serum or serum proteins does not sufficiently counteract the undesirable features of CPAs. For example, the combination of 10% DMSO with 50-90% fetal bovine serum (FBS) is a preferred freezing composition used in the state-of-the art. Yet, this preferred composition still leads to at least 20% cell death, even for cells that are considered relatively easy to freeze (Yong et al. Scientific Reports volume 5, Article number: 9596, 2015). Other limitations include the following:
- generally, serum supplements are considered inappropriate or undesirable for therapeutic applications;
- there is batch-to-batch variation in serum supplements, leading to inconsistent responses in cells;
- the level of endotoxin (lipopolysaccharide, LPS) is uncontrolled and this might hamper reproducibility of results;
- increasing ethical resistance towards the use of FBS in cryopreservation (Jochems et al. Altern Lab Anim. Mar-Apr 2002;30(2):219-27). FBS is harvested from foetuses taken from pregnant cows during slaughter and is therefore considered animal unfriendly.

In summary, it can be concluded that the use serum or other protein sources do not solve the problems most typically associated with CPAs.

### Thawing and cell stress and toxicity related to thawing

Even if toxicity can be minimized by adding a cryoprotectant before and during the freezing process, however, toxic effects may resume with thawing and the increasing temperatures associated with it. Post-thaw toxicity may have the same damaging potential at all liquid phases of the cryopreservation cycle but is commonly overlooked. Cells can be at least as sensitive to toxicity during thawing as they are on cooling, if not more, due to the fact that they are already stressed by the freeze/thaw cycle.

Despite the developments in the field of cryopreservation of biological materials for research and therapeutic applications, conventional CPAs have several undesirable features. There is an unmet need for methods that reduce the reliance on conventional CPAs, foremost DMSO or other permeating CPAs. In particular, there is need for cryopreservation methods that:
- reduce the amount of conventional CPAs needed during cryopreservation, thereby reducing the negative effects of CPAs such as cell toxicity, changes in cell functionality, and changes in inflammatory response;
- replace conventional CPAs entirely, thereby eliminating the negative effects of CPAs and furthermore rendering the cryopreserved cells safer and more effective in therapeutic applications, in particular cellular and tissue transplantation (ATMPs);
- allow cryopreservation of cell types for which conventional CPAs are deemed unsuccessful. These typically include slowly proliferating, terminally-differentiated cells, "hard-to-freeze" cells, or "non-freezable" cells, e.g. neuronal cells, cardiomyocytes, and/or other cell types;
- even if currently-used CPAs are beneficial during freezing, they may nevertheless be toxic during thawing due to the increased temperatures. There is a need of a method or cryopreservation formulation that allows bringing frozen cells to room temperature from a metabolically inactive state to an active state without causing much damage.

It is an object of the present invention to provide a solution to one or more of the problems associated with the cryopreservation and/or thawing of biological materials.

### SUMMARY OF THE INVENTION

The invention is as set out in the appended set of claims.

Surprisingly, the inventors found that the use of a relatively high concentration of collagen hydrolysate (preferably 20-50 wt.%) allows a reduction of the concentration of the cryoprotectant dimethylsulfoxide (i.e. DMSO) in cryopreservation. This strategy allows lowering the amount of dimethylsulfoxide, or even using no dimethylsulfoxide at all anymore, therefore it can remove several of the known drawbacks of dimethylsulfoxide.

The suitable cryopreservatives generally comprise 10% (v/v) or more dimethylsulfoxide, and is often considered as the "gold standard" in cryopreservation formulations. The inventors discovered that the combination of a relatively high concentration of collagen hydrolysate (preferably 20-50 wt.%) with a relatively low concentration of dimethylsulfoxide (namely 1 - 8.5 wt.%) may lead to highest cell recovery. The cells cryopreserved by this combination also showed improved viability (such as higher proliferation capacity and functionality) as compared to freezing in other cryopreservation formulations. Thus, not only does the cryopreservation formulation of the invention provide a higher recovery of cells as compared to other cryopreservation formulations, but also a higher viability of cells that are recovered. The collagen hydrolysate of the invention, in particular at the high concentrations described, allowed bringing frozen cells to room temperature from a metabolically inactive state to an active state apparently without causing damage. Hence, the collagen hydrolysate may exert beneficial effects on cells and/or reduce the undesirable effects of for instance dimethylsulfoxide in the thawing process.

At the preferred high concentration, the collagen hydrolysate have strong cryoprotective effects. For instance, the collagen hydrolysate may act as a cryoprotectant. In addition or alternatively, the collagen hydrolysate may further improve a cryoprotective effect of dimethylsulfoxide, reduce a toxic effect of dimethylsulfoxide, and/or maintain the genotype and phenotype of the cells. The collagen hydrolysate may eliminate the need for serum and serum-derived proteins that are commonly included reduce toxicity of dimethylsulfoxide. The cryopreservation formulation of the invention is particularly suitable for the preservation of biological material that are intended for human transplantation, such as an advanced therapy medicinal product (ATMP). The cryopreservation formulation of the invention is particularly suitable for the crypreservation cells which typically cannot be cryopreserved with dimethylsulfoxide alone and therefore can be considered as hard-to-freeze or non-freezable cells.

Overall, the best results were obtained when the amount of collagen hydrolysate exceeded 20 wt.%, particularly when it was 30 wt.% or 40 wt.%.

The current findings of the inventors are surprising, because they contradict the existing belief that high amounts of collagen hydrolysate should be avoided in cryopreservation. The state-of-the art for example points towards the use of no more than 15 wt.% collagen hydrolysate, in particular in combination with dimethylsulfoxide or other permeating cryoprotectants, to not impair the survival rate of cells after cryopreservation.

In an aspect, the present invention relates to a cryopreservation formulation comprising collagen hydrolysate and dimethylsulfoxide,
wherein the amount of collagen hydrolysate is 10-70 wt.% calculated on the weight of the cryopreservation formulation,
wherein the amount of dimethylsulfoxide is 1-8.5% (v/v) calculated on the volume of the cryopreservation formulation.

In embodiments, the present disclosure relates to a use of the cryopreservation formulation as disclosed herein for cryopreservation of one or more biological materials selected from the group consisting of an eukaryotic cell, a prokaryotic cell, a cell organelle, an extracellular vesicle, an organoid, a tissue, and an organ.

In embodiments, the present disclosure relates to a use of collagen hydrolysate in a cryopreservation formulation, wherein the collagen hydrolysate is used in an amount of 20-70 wt.%, calculated on the weight of the cryopreservation formulation.

In an aspect, the present invention relates to a method for cryopreservation of a biological material, the method comprising the step of providing the biological material in the cryopreservation formulation according to the invention, and reducing the temperature of cryopreservation formulation to below the freezing point of the cryopreservation formulation.

In an embodiment, the present disclosure relates a use of collagen hydrolysate for reducing the amount of dimethylsulfoxide in a cryopreservation formulation, wherein the collagen hydrolysate is provided in an amount of 20-70 wt.%, calculated on the weight of the cryopreservation formulation.

In embodiments, the present disclosure relates to a use of collagen hydrolysate for reducing toxicity and/or increasing viability in cells during thawing after cryopreservation, wherein the collagen hydrolysate is provided in a cryopreservation formulation in an amount of 20-70 wt.%, calculated on the weight of the cryopreservation formulation.

### DETAILED DESCRIPTION OF THE INVENTION

The cryopreservation formulation of the invention pertains to a composition comprising collagen hydrolysate in an amount of 10-70 wt.%, preferably in an amount of 20 - 70 wt.%, calculated on the weight of the cryopreservation formulation.

The term "collagen" in the context of the current invention means an amino acid sequence comprising a repeating (Gly-X-Y) sequence, preferably comprising at least 2, 3, 4, 5, 10, 20, 50, 100, 200, 300, or 400 sequences containing the sequence Gly-X-Y, where X and Y are an amino acid residue independently chosen from each other, but X and/or Y are more preferably proline. The "collagen" preferably has a sequence found in native collagen in one or more animal species. In addition or alternatively, the "collagen" can mean a full-length sequence or fragment or subunit thereof of (native) collagen, preferably one or more of collagen types I - XXVII, more preferably one or even more of type I, II, III, V, or X collagen, even more preferably one or more of type I, II or III collagen. For example, the term "collagen" may refer to an alpha-1(I), alpha-2(I), alpha-1(ll) or alpha-1(lll) chain, or a fragment thereof. The term "collagen" encompasses the triple helix structure as formed by three subunits as present in native collagen.

The term "collagen hydrolysate" the context of the current invention means a mix of short chains of amino acids (di-, tri, oligopeptides, polypeptides) derived from (partial) hydrolysis from native (full-length) collagen, such as by enzymatic hydrolysis. The degree of hydrolysis has an impact on the average molecular weight (expressed in Dalton, Da) of the final product. The term "collagen hydrolysate" may be used interchangeably and synonymous with the terms "hydrolysed collagen" or "collagen peptide". The "collagen hydrolysate" in the context of the current invention encompasses collagen which is subjected to hydrolysis or partial hydrolysis.

In an embodiment, the hydrolysis is alkali hydrolysis. In an embodiment, the hydrolysis is acid hydrolysis. In an embodiment, the hydrolysis is enzymatic hydrolysis. The collagen hydrolysate can be one or more of enzyme-hydrolysed, alkali hydrolysed and acid-hydrolysed collagen hydrolysate. In addition or alternatively, the collagen hydrolysate can be obtained by one of the following processes or a combination thereof: alkali hydrolysis, acid-hydrolysis and enzyme- hydrolysis.

The "collagen hydrolysate" in the context of the current invention may be produced from a collagen-containing material in a one-step process or via an intermediate gelatin stage (i.e. thus "hydrolysed gelatin" is obtained). The term "collagen hydrolysate" thus encompasses hydrolysed gelatin (i.e. hydrolyzed gelatin). The term "gelatin" as used herein means a (irreversible) form of collagen as obtained by partial hydrolysis of collagen. Depending on the process used, two types of gelatin, namely type A (acid hydrolysis) and type B (alkaline hydrolysis) are generally obtained. In the context of the current invention, the "gelatin" can refer to either Type A or Type B gelatin, or a combination thereof. Depending on the physical and chemical methods of the partial hydrolysis, the molecular weight of the peptides can fall within a broad range (e.g. 10-95 kDa). The partial hydrolysis provides the gelatin the ability to hold water and its gelling capacity, typically distinguishing it from "collagen peptide" or "hydrolysed collagen" i.e. products obtained by full hydrolysis of collagen. The term "hydrolysed gelatin" in the context of the current invention means a product obtained by the hydrolysis of gelatin, and which leads to a lower molecular weight of the gelatin. The hydrolysis reaction as to obtain hydrolyzed gelatin involves the rupturing of one or more of the peptide linkings with the addition of 1 molecule of water. Hydrolyzed gelatin distinguishes from gelatin among others, in terms gelling capacity, i.e. hydrolyzed gelatin has reduced/no gelling capacity and gelatin has gelling capacity. The gelatin may be hydrolyzed by acids (hydrogen ions), i.e. to obtain "acid-hydrolyzed gelatin". The gelatin may be hydrolyzed by alkali (hydroxyl ions), i.e. to obtain "alkali-hydrolysed gelatin. The gelatin may be hydrolyzed by one or more enzymes (e.g. pepsin, trypsin), i.e. to obtain "enzyme-hydrolysed gelatin". The terms "hydrolyzed" and "hydrolysed" can be used interchangeably in the context of the current invention.

In a preferred embodiment, the collagen hydrolysate is hydrolysed gelatin.

The collagen or collagen hydrolysate in the context of the current invention may include proteins or peptides that are synthesized artificially, such as recombinantly or by chemical synthesis. Recombinant synthesis encompasses that a protein or peptides is encoded by recombinant DNA that is expressed in an expression system. The expression system for the recombinant peptide can be cells such as a bacterial cell (e.g., Escherichia coli, Bacillus subtilis species), yeast cell [e.g. Saccharomyces cerevisiae, Pichia pastoris or Ogataea angusta (Hansenula polymorpha), Candida bodini], fungal cell (e.g. Aspergillus oryzae, Aspergillus niger, Trichoderma reesei), mammalian cell (e.g. a CHO cell, a HeLa cell, a HEK293 cell, NS0, Sp2/0), insect cell and plant cell (e.g. tobacco, cereal, legume, fruit, vegetable).

In various embodiments of the current invention, the collagen hydrolysate may be produced by the enzymatic hydrolysis or partial enzymatic hydrolysis of collagen, wherein the enzyme used for this purpose may be one or more selected from the group consisting of serine protease, alkaline protease, neutral protease, flavor protease, complex protease, thiol protease, bromelain, metalloprotease, protease, carboxypeptidase, pepsin, chymotrypsin, trypsin, cathepsin K, chymotrypsin, papain, and subtilisin.

In an embodiment, the hydrolysis, preferably the enzymatic hydrolysis, is performed at pH 5-8, preferably pH 6-7. In an embodiment, the hydrolysis, preferably the enzymatic hydrolysis is performed at a temperature of 55-70 °C, preferably 60-65 °C. In an embodiment, the hydrolysis, preferably the enzymatic hydrolysis, is performed for 3-8 hours, preferably 4-7 hours, more preferably 5-6 hours.

The collagen hydrolysate in the context of the current invention may be derived from one or more types of collagen selected from collagen types I - XXVII, preferably one or more of type I, II, III, V, or X collagen, more preferably one or more of type I, II or III collagen. In addition or alternatively, the collagen hydrolysate as disclosed herein may be a mixture of two or more types of collagen, preferably two one or more of type I, type II, and type III collagen.

The collagen as disclosed herein may be derived from any one or more animals or species of animals, such as bovine (species), pig (species), chicken and fish (species).

In an embodiment, the collagen is derived from a cow. In an embodiment, the collagen as is derived from a pig. In an embodiment, the collagen is derived from a fish. In an embodiment, the collagen is derived from a chicken. In various embodiments, the collagen is a mixture of collagen from different sources, such as collagen originating from multiple animal species and/or collagen originating from different tissues. For example, the collagen as disclosed herein may be a mixture of two or more collagens chosen from the group consisting of fish collagen, porcine collagen, chicken collagen, and bovine collagen.

The collagen in the context of the current invention may be derived from one or more tissues selected from the group consisting of skin, scale, antler, protrusions (e.g. humps) horns, head, brain, neck, ear, eye, nose, tongue, lip, mouth, oesophagus, trachea, sternum, larynx, bronchi, limbs, feet, toes, palms, claws, bones, cartilage, bone marrow, joints, membranes, hind, ligaments, tendon, rib, diaphragm, muscle, skeletal muscle, smooth muscle, intestine, blood vessels, bladder, stomach, aorta, heart, liver, kidney, chest, lung, spleen, pancreas, egg, sperm, testis, ovary, nerve, gallbladder, and belly. The term "skin" as disclosed herein encompasses "hide", i.e. meaning the outer covering of large animals such as from bovine (species) or any other large animals. The terms "skin" and "hide" may herein be used interchangeably, and may refer the outer coverage of an animal, irrespective of size.

In a preferred embodiment, the collagen as taught herein is derived from skin and/or skin connective tissue. In preferred embodiment, the collagen as taught herein is derived from cartilage. In a preferred embodiment, the collagen as taught herein is derived from bone. In a preferred embodiment, the collagen as taught herein is derived from sternum. The collagen as disclosed herein may be a mixture of collagens derived from two or more tissues and/or two or more animals. In an embodiment, the collagen as disclosed herein is a mixture of two or more collagens selected from the group consisting skin collagen, cartilage collagen, sternum cartilage and bone collagen.

The term "cryopreservation formulation" in the context of the current invention means a formulation suitable and/or intended for use in cryopreservation of cells. The formulation is preferably a liquid formulation, or at least a formulation in which biological materials including cells can be suspended. The term "cryopreservation" as used herein means the cooling, preferably freezing of a biological material, preferably with the aim of allowing storage of the biological material and/or saving the biological material for future use. The cryopreservation most typically, and preferably in the context of the current invention, achieves a temperature of in the order of -10 to -30 °C, more preferably in the order of -70 to -90 °C (e.g. using solid carbon dioxide) or in the order of -180 to -220 °C (e.g. using liquid nitrogen). The term "cryopreservation" can be used interchangeably with the terms "cryoprotection" or "cryobanking" in the context of the present invention. The term "cryopreservation" as used herein encompasses the cooling of the material to above freezing point, for example to avoid formation of ice crystals. The term "cryopreservation" encompasses slow freezing. "Slow freezing" means that the temperature is gradually reduced, most typically 0.2 - 5 °C (e.g. 0.5 - 2 °C) per minute on average until it reaches the final storing temperature. This means that the biological material is cooled over the course of several hours (e.g. to achieve a temperature of around -196 °C). "Slow freezing" can be used interchangeably with the terms "controlled-rate freezing" or "slow programmable freezing (SPF)" in the context of the present invention. The term "cryopreservation" as used herein encompasses "vitrification" (also called flash-freezing), which involves rapid cooling with the aim of preventing the formation of ice crystals and thereby help prevent cryopreservation damage. The processes of "slow freezing" and "vitrification" are well known in the art, e.g. as described in the review article by Son et al. (Expert Rev Med Devices 2009 Jan;6(1):1-7).

In the context of the current invention, the term "cryopreservation" preferably encompasses the steps of incorporating the biological material in a cryopreservation formulation the freezing, and the thawing of the cells after freezing. In the context of the current invention, a formulation that leads to improvement in cells during thawing are considered to improve the cryopreservation in general. The current invention also further pertains to the thawing of biological materials after freezing. In particular, the present inventors found that collagen hydrolysate at the appropriate concentration allow bringing frozen cells to room temperature from a metabolically inactive state to an active state, apparently without causing damage. Hence, the collagen hydrolysate may exert beneficial effects during thawing and/or reduce the negative (toxic) effects of dimethylsulfoxide during thawing.

In an embodiment of the present disclosure, the use of the collagen hydrolysate and/or the cryopreservation formulation as disclosed herein pertains to a use in reducing toxicity and/or increasing viability in cells during thawing after cryopreservation, wherein the collagen hydrolysate is preferably provided in a cryopreservation formulation in an amount of 20-70 wt.%, calculated on the weight of the cryopreservation formulation. In a preferred embodiment of the present disclosure, reducing the toxicity and/or increasing the viability in the cells is by reducing one or more toxic effects of a non-permeating and/or a permeating cryoprotectant in the cells during the thawing. In a preferred embodiment of the present disclosure, reducing the toxicity and/or increasing the viability in the cells is by reducing one or more toxic effects of a permeating cryoprotectant in the cells during the thawing, wherein the permeating cryoprotectant is dimethylsulfoxide.

The method of the invention pertains to providing a biological material in a cryopreservation formulation according to the invention and reducing the temperature of cryopreservation formulation to below the freezing point of the cryopreservation formulation. The freezing point may be dependent on the composition of a liquid cryopreservation formulation, and is defined as the temperature at which the liquid cryopreservation formulation becomes a solid at normal atmospheric pressure. In addition or alternatively, considering that the cryopreservation formulation can be aqueous, the freezing point is preferably 0°C or near 0°C (e.g. -10, -9, -8, - 7, -6, -5, -4, -3, -2 -1, 1, 2, 3, 4, 5, 6, 7, 8, 9, or 10 °C).

In an embodiment, the cryopreservation formulation with biological material is directly frozen, meaning that the freezing rate is not controlled, thus excluding the use of a programmable and/or slow-freezing.

The cryopreservation formulation may comprise at least 10 wt.%, or 15 wt.%, or 20 wt.%, or 22.5 wt.%, or 25 wt.%, or 27.5 wt.%, or 30 wt.%, or 32.5 wt.%, or 35 wt.%, or 37.5 wt.%, or 40 wt.%, or 45 wt.%, or 50 wt.% collagen hydrolysate, calculated on the weight of the cryopreservation formulation. In addition or alternatively, the cryopreservation formulation may comprise no more than 70 wt.%, or 65 wt.%, or 50 wt.%, or 45 wt.%, or 40 wt.%, or 37.5 wt.%, or 35 wt.%, or 32.5 wt.%, or 30 wt.%, or 27.5 wt.%, or 25 wt.%, or 22.5 wt.%, or 20 wt.% collagen hydrolysate, calculated on the weight of the cryopreservation formulation. In a preferred embodiment, the cryopreservation formulation comprises 20 - 70 wt.%, preferably 20 - 50 wt.% collagen hydrolysate, more preferably 25 - 40 wt.%, even more preferably 27.5 - 35 wt.% collagen hydrolysate, calculated on the weight of the cryopreservation formulation.

The cryopreservation formulation, e.g. for the use as described herein, may comprise at least 1% v/v, or 1.25% v/v, or 1.5% v/v, or 1.75% v/v, or 2% v/v, or 2.25% v/v, or 2.5% v/v, or 2.75% v/v, or 3% v/v, or 3.25% v/v, or 4% v/v, or 4.25% v/v, or 4.5% v/v, or 4.75% v/v, or 5% v/v, or 5.25% v/v, or 5.25% v/v, or 5.5% v/v, or 5.75% v/v, or 6.0% v/v, or 6.25% v/v, or 6.5% v/v, or 7% v/v, or 7.5% v/v, or 8% v/v dimethylsulfoxide, calculated on total volume of the cryopreservation formulation. In addition or alternatively, the cryopreservation formulation may comprise no more than 8.5% v/v, or 8% v/v, or 7.5% v/v, or 7% v/v, or 6.5% v/v, or 6.25% v/v, or 6% v/v, or 5.75% v/v, or 5.5% v/v, or 5.25% v/v, or 5% v/v, or 4.75% v/v, or 4.5% v/v, or 4.25% v/v, or 4% v/v, or 3.75% v/v, or 3.5% v/v, or 3.25% v/v, or 3% v/v, or 2.75% v/v, or 2.5% v/v dimethylsulfoxide, calculated on the total volume of the cryopreservation formulation.

In a preferred embodiment, the cryopreservation formulation, e.g. for the use as described herein, comprises more than 20 wt.%, preferably more than 22.5 wt.%, even more preferably more than 25 wt.% collagen hydrolysate, calculated on the weight of the cryopreservation formulation. In a preferred embodiment, the cryopreservation formulation, e.g. for the use as described herein, comprises less than 50 wt.%, preferably less than 45 wt.%, even more preferably less than 40 wt.% collagen hydrolysate, calculated on the weight of the cryopreservation formulation.

The skilled person knows how to convert between the wt.% and volume % amounts of DMSO, based on the known density of DMSO (1.1 g/cm³) and the density of the aqueous medium that the DMSO is present in (typically density ~1.0 g/cm³). For example, a 10% (v/v) DMSO concentration calculated on the volume of an aqueous formulation is equal to 10.9 wt.% DMSO calculated on the total weight of the formulation. The same conversion applies to for example a DMSO concentration of 0.5% v/v (i.e. 0.55 wt.%), 1.0% v/v (i.e. 1.10 wt.%), 1.5% v/v (i.e. 1.65 wt.%), 2.0% v/v (i.e. 2.2 wt.%), 2.5% v/v (i.e. 2.74 wt.%), 3.0% v/v (i.e. 3.29 wt.%), 3.5% v/v (i.e. 3.84 wt.%), 4.0% v/v (i.e. 4.38 wt.%), 4.5 v/v (i.e. 4.93 wt.%), 5.0% v/v (i.e. 5.24 wt.%), 5.5% v/v (i.e. 6.02 wt.%), 6.0% v/v (i.e. 6.56 wt.%), 6.5% v/v (i.e. 7.10 wt.%), 7.0% v/v (i.e. 7.65 wt.%), 7.5% v/v (i.e. 8.16 wt.%), 8.0% v/v (i.e. 8.73 wt.%), 8.5% v/v (i.e. 9.27 wt.%), 9.0% v/v (i.e. 9.81 wt.%), 9.5% v/v (i.e. 10.35 wt.%), 10% v/v (i.e. 10.90 wt.%), 12.5% v/v (i.e. 13.58 wt.%), 15% v/v (i.e. 16.26 wt.%).

In an embodiment, the current disclosure pertains to a use of a collagen hydrolysate disclosed herein in cryopreservation of a biological material, wherein the biological material is preferably selected from the group consisting of an eukaryotic cell, a prokaryotic cell, a cell organelle, an extracellular vesicle, an organoid, a tissue, and an organ. In a preferred embodiment, the collagen hydrolysate in the use described herein is present in an amount of 10-70 wt.%, preferably 20-60 wt.%, more preferably more than 20 wt.%, calculated on the total weight of the cryopreservation formulation. In a preferred embodiment, the collagen hydrolysate in the use described herein is (essentially) free of one or more of a further cryoprotectant, serum and a serum protein, more preferably free of a further permeating cryoprotectant.

The method of the invention makes use of a cryopreservation formulation with an amount of collagen hydrolysate of 10-70 wt.%, preferably 20-60 wt.%, more preferably more than 20 wt.%, calculated on the total weight of the cryopreservation formulation. In a preferred embodiment, the method of the invention makes use of a cryopreservation formulation which is (essentially) free of one or more of a further cryoprotectant, serum and a serum protein, more preferably free of a further permeating cryoprotectant.

In an embodiment, the method and/or use of the present disclosure makes use of collagen hydrolysate as cryoprotectant in absence of one or more of a further cryoprotectant, serum and a serum protein, more preferably in absence of DMSO.

In the embodiment wherein the collagen hydrolysate in used in absence of one or more of a further cryoprotectant, serum and a serum protein, the collagen hydrolysate preferably is hydrolyzed gelatin, as this appears to lead to highest viability after cryopreservation, notwithstanding that though other forms of collagen hydrolysate can also suitable for the invention.

In the embodiment wherein the collagen hydrolysate in used in absence of one or more of a further cryoprotectant, serum and a serum protein, the collagen hydrolysate preferably has a molecular weight of 3500 - 7500 Da, preferably 4000 - 6000 Da, more preferably 4500 - 5500 Da, as this appear to lead to highest viability after cryopreservation, notwithstanding that other forms of collagen hydrolysate can also suitable for the invention.

In the embodiment wherein the collagen hydrolysate in used in absence of one or more of a further cryoprotectant, serum and a serum protein, the amount of collagen hydrolysate is preferably more than 20-50 wt.%, preferably 22.5-45 wt.%, more preferably 25-40 wt.%, even more preferably 27.5 - 35 wt.%, calculated on the total weight of the formulation, as this appears to lead to highest viability after cryopreservation, notwithstanding that other forms of collagen hydrolysate can also suitable for the invention.

In an embodiment, the use of the present disclosure pertains to use of collagen hydrolysate for reducing the amount of dimethylsulfoxide in a cryopreservation formulation, wherein the collagen hydrolysate is preferably provided in an amount of 20-70 wt.% calculated on the weight of the cryopreservation formulation. For instance, the amount of dimethylsulfoxide may be reduced due to collagen hydrolysate, while nevertheless achieving at least 75%, or at least 80%, or at least 85%, or at least 90%, or at least 95%, or at least 99% cell recovery.

In an embodiment, cryopreservation of a biological material, preferably cells, in the cryopreservation formulation of the invention, leads to similar or higher cell recovery (and/or lower cell death) and/or cell viability, as compared to the same cryopreservation formulation, but without the collagen hydrolysate. For instance, the combination of 25 wt.% collagen hydrolysate and 5 wt.% DMSO may lead to similar or higher cell recovery (and/or lower cell death) and/or cell viability as compared to 5 wt.% DMSO without collagen hydrolysate.

In an embodiment of the present disclosure, the use of collagen hydrolysate in cryopreservation of a biological material, preferably cells, allows a lower amount of DMSO to be used, while leading to similar or higher cell recovery (and/or lower cell death) and/or cell viability. For instance, the combination of 25 wt.% collagen hydrolysate and 5 wt.% DMSO may lead to similar or higher cell recovery (and/or lower cell death) and/or cell viability compared to use of 10 wt.% DMSO without collagen hydrolysate. "A lower amount of DMSO" can for in stance be a reduction in the amount of DMSO by at least 10%, or at least 20%, or at least 30%, or at least 40%, or at least 50%, or at least 60%, or at least 70%, or at least 80%, or at least 90%, or at least 95%, or at least 99% and/or a reduction to a any of the preferred DMSO concentrations as disclosed herein.

The "cell recovery" and/or "cell death" in the context of the current invention is preferably determined after slow freezing of cells and determining the percentage of live and dead cells after thawing. The skilled person knows that the slow freezing and thawing may be dependent on the type of cells, and can adapt the protocol accordingly. The cell recovery after thawing can be determined by counting the live cells and comparing it to the number of frozen cells, e.g. using a stain that selectively stains dead cells such as trypan blue. The % of cell death can be determined by comparing the live cells to the number of frozen cells. In addition or alternatively, the live and dead cells can be determined using a commercial assay, such as a Live Dead assay, which uses a mixture of two fluorescent dyes that differentially label live and dead cells, and measuring live and dead cells using flow cytometry or fluorescent microscopy.

In the context of the current invention, the term "live cell" means a cell with an intact cell membrane, e.g. defined by showing that a (DNA-binding) cell membrane-impermeable dye cannot enter the cell. In the context of the current invention, the term "dead cell" means a cell that is not a live cell.

In a context of the current invention, the "cell viability" is preferably determined according to one or more of the cellular metabolic activity, the cell proliferation, and the (adenosine triphosphate) ATP concentration, i.e. a higher cellular metabolic activity, a higher cell proliferation, and/or a higher ATP concentration may be an indicator of higher cell viability.

The cellular metabolic activity can for instance be measured with an assay based on a tetrazolium salt (e.g. MTT, XTT) or Alamar Blue. The cell proliferation can for instance be measured with an assay for a cell proliferation marker or a cell cycle regulatory marker such as the Ki-67, proliferating cell nuclear antigen (PCNA), topoisomerase IIB, or phosphorylated histone H3. ATP can for instance be detected using the bioluminescent luciferase and its substrate, luciferin.

In an embodiment, the use of the present invention pertains to use of collagen hydrolysate for reducing the amount of dimethylsulfoxide in a cryopreservation formulation, wherein the collagen hydrolysate is preferably provided in an amount of 20-70 wt.%, calculated on the weight of the cryopreservation formulation.

The collagen hydrolysate may have an average molecular weight in the range of 500 Da - 25,000 Da, e.g. 1000 Da to 15000 Da or 2000 Da to 10000 Da. The collagen hydrolysate may have an average molecular weight of at least 500 Da, or 600 Da, or 700 Da or 800 Da, or 900 Da, or 1000 Da or 1100 Da, or 1200 Da, or 1300 Da, or 1400 Da, or 1500 Da, or 1750 Da, or 2000 Da, or 2250 Da, or 2500 Da, or 2750 Da, or 3000 Da, or 3250 Da, or 3500 Da, or 4000 Da, or 4500 Da, or 5000 Da, or 5500 Da. In addition or alternatively, the collagen hydrolysate may have an average molecular weight of no more than 10000 Da, or 9500 Da, or 9000 Da, or 8750 Da, or 8500 Da, or 8250 Da, or 8000 Da, or 7750 Da, or 7500 Da, or 7250 Da, or 7000 Da, or 6750 Da, or 6500 Da, or 6250 Da, or 6000 Da, or 5750 Da, or 5500 Da, or 5250 Da, or 5000 Da, or 4500 Da, or 4000 Da. In a preferred embodiment, the collagen hydrolysate has an average molecular weight of 500-10000 Da, preferably 1000-8000 Da, more preferably 2000 - 7000 Da, even more preferably 3000 - 6000 Da.

The average molecular weight in the context of the current invention is preferably the weight-average molecular weight. A preferred way of measuring the (average) molecular weight and/or molecular weight distribution of collagen hydrolysate or gelatin is by high performance size exclusion chromatography (HPSEC). The following protocol is a preferred HPSEC protocol:
The Agilent HPLC, 1260 Infinity series (G1316A, G1329B, G1311C, G1315D) with a TSKgel SWXL precolumn und a G2000SWXL column (Tosoh Bioscience) is used. Analysis is performed with the WinGPC software (PSS). The eluent is 100 mM phosphate buffer pH 5.3. Samples are eluted from the column (e.g. 0.5 mL/min, isocractic) and monitored with UV detection (e.g. 214 nm, analysis time: 40 min per injection + 180 min equilibration). Calibration is performed with the Narrow Calibration Standard (Low FILK).

The inventors found that it is beneficial in the context of the current invention if no or minimal LPS (endotoxin) is present, in particular when a centrifugation washing step is performed. Having no or low LPS may make the centrifugation washing step further redundant. It was furthermore found that the presence of LPS during cryopreservation may induce an undesirable toxic and/or pro-inflammatory response in cells. The use of collagen hydrolysate may in addition or alternatively also protect against the undesirable effects of LPS in the cryopreservation formulation.

The collagen hydrolysate preferably has an endotoxin level of no more than 10000 EU (i.e. endotoxin units) per g collagen hydrolysate or per mL cryopreservation formulation, preferably no more than 1000 EU (e.g. no more than 500, 400, 300, or 200 EU), more preferably no more than 100 EU (e.g. no more than 90, 80, 70, 60, 50, 40, or 30, or 20 EU), even more preferably no more than 10 EU (e.g. no more than 9, 8, 7, 6, 5, 4, 3, 2, 1 EU), most preferably no more than 1 EU, all per g collagen hydrolysate or per mL cryopreservation formulation. In addition or alternatively, the collagen hydrolysate may have an endotoxin level of at least 1 EU per g collagen hydrolysate or per mL cryopreservation formulation, preferably at least 10 EU, such as at least 20 EU (e.g. at least 30, 40, 50, 60, 70, 80, 90,100 or 150 EU), more preferably at least 100 EU (e.g. at least 200, 300, 400, 500, 600, 700, 800, 900, 1000, or 1500 EU), even more preferably at least 1000 EU (e.g. at least 3000, 4000, 5000, 6000, 7000, 8000, 9000, or 1000 EU), most preferably at least 10000 EU, all per g collagen hydrolysate or per mL cryopreservation formulation.

In a preferred embodiment, the collagen hydrolysate has an endotoxin level of no more than 10000 EU/g, preferably no more than 1000 EU/g, more preferably of more than 100 EU/g, most preferably no more than 10 EU/g, calculated on the weight of the collagen hydrolysate.

In a preferred embodiment, the cryopreservation formulation has an endotoxin level of less than 2500 EU/ml, preferably less than 250 EU/ml, more preferably less than 25 EU/ml, even more preferably less than 2.5 EU/ml, most preferably less than 1 EU/ml, calculated on the volume of the cryopreservation formulation. For example, the cryopreservation formulation may have an endotoxin level 0.5 - 2500 EU/ml, or 1 - 1000 EU/ml, or 5 - 500 EU/ml or 10 - 250 EU/ml, calculated on the volume of the cryopreservation formulation.

In an embodiment, the collagen hydrolysate is endotoxin-free. In an embodiment, the cryopreservation formulation is endotoxin-free. The term "endotoxin-free" can mean absence of endotoxin and/or can mean "essentially free of endotoxin".

The term "free of" can mean the same as "essentially free of". The term "essentially free" encompasses that the amount of a substance or compound is not measurable according to a standard technique in the field and/or is below a certain threshold, preferably below 0.1 wt. %, more preferably below 0.01 wt.%, even more preferably below 0.001 wt.%, most preferably below 0.0001 wt.%. In the context of the current invention, "essentially free" may mean that the endotoxin level is below 10, preferably below 1, more preferably below 0.1, even more preferably below 0.01, most preferably below 0.001 (all in EU/g or EU/ml). The wordings "free" or "essentially free" encompass that a substance or compound is completely absent (e.g. 0 wt.%). The terms "in absence", "free" and "essentially free" can be used interchangeably in the context of the current invention.

Widely-used and preferred methods for measuring endotoxin level (e.g. for determining EU/g or EU/ml) in the context of the current invention are the Limulus Amebocyte Lysate (LAL) test or the recombinant Factor C (rFC) test, which the skilled person is familiar with.

In an embodiment, the cryopreservation formulation is free of one or more of a further cryoprotectant, serum and a serum protein.

The term "cryoprotectant" in the context of the current invention means any substance or compound used in cryopreservation of a biological material and which protects against (i.e. at least reduces) freezing damage in the biological material such as by reducing ice crystal formation. The terms "cryoprotectant", "cryopreservant", "cryopreservative" and "antifreeze (agent)" may be used interchangeably in the context of the current invention. A cryoprotectant is typically referred to as an "antifreeze (agent)" when applied outside cryobiology. In the context of the current invention, a substance or compound is consider to be a cryoprotectant if a solution comprising the substance or compound vitrifies after cooling (e.g. at -80 °C or in liquid nitrogen). "Vitrification" in the context of the current invention means the formation of solid water with an irregular, amorphous structure. Vitrification is preferably determined by preparing a solution comprising the substance or solution and cooling for 30 min at -80 °C or in liquid nitrogen. The cooled solution is considered to be vitrified if it becomes transparent (and not milky), e.g. if the transparency is equal or higher than as observed for a reference (well-known) cryoprotectant, such as a permeating or a non-permeating cryoprotectant as disclosed herein. For example, the transparency of a solution comprising 20% (w/v) collagen hydrolysate may be compared to dimethylsulfoxide (10% v/v), ethylene glycol (1 M), propylene glycol (1 M), or glycerol (20% v/v) as reference. Dose-response curve can be obtained to determine optimal vitrification (for collagen hydrolysate).

The cryoprotectant in the context of the current invention encompasses both permeating and non-permeating cryoprotectants. The term "permeating cryoprotectant" means a cryoprotectant that has the capacity to penetrate into cells and thereby may provide intracellular protection against freezing damage. A permeating cryoprotectant is able to cross biological membranes. The term "non-permeating cryoprotectant" means a cryoprotectant that (largely) does not have the capacity to penetrate into cells and/or (largely) does not require the penetration into cells to mediate the cryoprotective effect. Non-permeating cryoprotectants generally induce vitrification by the same mechanism as permeating cryoprotectants, but extracellularly. In the context of the current invention, the cell permeability of a compound or substance is preferably defined by an increase in cell volume after exposing cells to a solution comprising the compound or substance. Accordingly, the substance or compound can for example be classified as either a permeating or a non-permeating cryoprotectant or cryopreservant. The permeation speed into the cells can be determined by measuring the cell volume at specific time intervals after exposure of the cells to the solution. A preferred protocol is as described by Eto et al (Cryobiology. 2014 Feb;68(1):147-51). Next, an example is provided to determine the possible cell-permeability of collagen hydrolysate:
Cells (e.g. 3T3 fibroblast cell line) are exposed to a solution with 20% w/v collagen hydrolysate at 25 ± 0.5 °C. Solutions comprising permeating dimethylsulfoxide (10% v/v), ethylene glycol (1 M), propylene glycol (1 M), or glycerol (20% v/v) are used as reference and the cell diameter is measured (control), 30, 60, 120, 180, 240, and 300 s later. The volume is calculated with the formula V = S^{3/2} (S: relative cross-sectional area; V: relative volume, S = πab; a: radius of the long axis; b: radius of the minor axis) and the ratio of the volume at each time point is calculated with the control volume. The collagen hydrolysate is considered to permeate the cells if the average relative volume for the collagen hydrolysate solution at least matches the average volume for a reference (for one or more of the measured time points). The cells exposed to the collagen hydrolysate solution may initially shrink, and thereafter recover (once the collagen hydrolysate enters the cell). Therefore, the volume change is preferably calculated starting from the lowest V value.

It was found that the collagen hydrolysate may further improve a cryoprotective effect of dimethylsulfoxide, reduce a toxic effect of dimethylsulfoxide, and/or maintain the genotype and phenotype of the cells (e.g. by DNA sequencing, methylation, cell surface marker expression, cell differentiation assays or other functional tests).

In an embodiment, the collagen hydrolysate is a cryoprotectant. In an embodiment, the collagen hydrolysate is a permeating cryoprotectant. In an embodiment, the collagen hydrolysate is a non-permeating cryoprotectant. In an embodiment, the collagen hydrolysate is not a cryoprotectant. In an embodiment, the collagen hydrolysate improves the effect and/or reduces the toxicity of a (further) cryoprotectant, preferably a permeating cryoprotectant, more preferably dimethyl sulfoxide.

Without being bound by theory, the present inventors found that the collagen hydrolysate may have one or more mechanisms (in combination with DMSO) in improving cell recovery and viability after cryopreservation, such as providing cell-binding sites, water-binding/absorption, influencing osmotic pressure, influencing intracellular water content and ice crystal formation. Hence, collagen hydrolysate may provide one or more multiple mode of actions in context of the current invention.

In a preferred embodiment, the cryopreservation formulation comprises one or more of a further cryoprotectant, serum, and a serum protein.

In an embodiment, the permeating cryoprotectant is one or more selected from the group consisting of dimethyl sulfoxide, ethylene glycol, propylene glycol, and glycerol.

If dimethylsulfoxide is present in the formulation, the term "further cryoprotectant" refers to any cryoprotectant other than dimethylsulfoxide. If dimethylsulfoxide is not present in the formulation, the term "further cryoprotectant" may refer to any cryoprotectant, including dimethylsulfoxide.

In an embodiment, the non-permeating cryoprotectant is one or more selected from the group consisting of a polyethylene glycol, a sugar (e.g. sucrose, dextrose, raffinose, trehalose, lactose), polyvinylpyrrolidone, and methylcellulose.

It was found that addition of collagen hydrolysate may eliminate the need for serum and serum-derived proteins that are commonly used in the art to reduce toxicity of cryoprotectants. Without being bound by theory, the collagen hydrolysate may protect the cell membrane against shear stresses and/or counteract the undesirable features of dimethylsulfoxide or other cryoprotectants. Replacing serum and serum-derived proteins may additionally or alternatively reduce batch-to-batch variations, inconsistent responses in cells, and resistance in use with respect to ethical or therapeutically considerations.

The term "serum" as disclosed herein encompasses human and animal serum, including but not limited to fetal bovine serum (FBS), fetal calf serum (FCS), or newborn bovine serum (NBS), newborn calf serum. The term "serum" as disclosed herein encompasses heat-inactivated serum. The term "serum protein" in the context of the current invention encompasses human serum proteins and animal serum proteins, preferably human serum albumin (HSA) or bovine serum albumin (BSA).

In this invention, salt may or may not be present in the cryopreservation formulation

In an embodiment, the cryopreservation formulation is (essentially) salt-free.

In an embodiment, the cryopreservation formulation is (essentially) free of added salts.

In an embodiment, the cryopreservation formulation is animal-free, meaning that it contains no animal-derived ingredients, for instance if the collagen (hydrolysate) or gelatin (hydrolysate) is recombinantly or synthetically obtained.

In an embodiment, the cryopreservation formulation comprises one or more of a permeating glycol in an amount of 0.1 - 20 wt.%, preferably 0.2 - 10 wt. %, more preferably 0.5 - 5 wt. % , even more preferably 1 - 2.5 wt.%, calculated on the weight of the cryopreservation formulation. The "permeating glycol" as used herein preferably is ethylene glycol and/or propylene glycol.

In an embodiment, the cryopreservation formulation comprises one or more sugars in amount of 0.1 - 20 wt.%, preferably 0.2 - 10 wt.%, more preferably 0.5 - 5 wt.%, even more preferably 1-2.5 wt.%, calculated on the weight of the cryopreservation formulation.

The "sugar" in the context of the current invention is preferably one or more selected of the group consisting of sucrose, dextrose, raffinose, trehalose and lactose.

In the context of the current invention, the cryopreservation formulation is suitable for the cryopreservation of one or more biological materials, such as one or more selected from the group consisting of cells (eukaryotic or prokaryotic), multicellular eukaryotes (e.g. (Nematode) worms), organoid (e.g. intestinal organoid, lung organoid, embryonic organoid, kidney organoid, epithelial organoid), microbiological culture (e.g. fungi, bacteria), blood (e.g. umbilical cord blood), semen, thrombosomes, tissues (e.g. living tissues or non-living tissues like tumours and histological cross sections), ovarian tissue, skin tissue, musculoskeletal tissue (e.g. bone, cartilage, tendon), testicular tissue, sperm, oocytes, embryos, organ (e.g. liver, heart, kidney, skin, lung, pancreas, intestine), cellular vesicle (e.g. extracellular vesicles including exosomes), organoids, plant material (e.g. plant seeds, shoots tips, dormant buds, moss plant).

The biological material is preferably mammalian, more preferably human.

In a preferred embodiment, the use of the disclosure pertains the cryopreservation of one or more biological materials selected from the group consisting of an eukaryotic cell, a prokaryotic cell, a cell organelle, an extracellular vesicle (EV), an organoid, a tissue, and an organ.

3D organoids closely mimic the in vivo situation comprising proliferative stem and progenitor cells as well as differentiated cell types including absorptive enterocytes, secretory mucus producing, paneth or enteroendocrine cells. The cell types are localized in distinct proliferation (crypt) or differentiation (villus) zones in the overall organoid architecture. 3D organoids represent structurally closed systems characterized by the formation of a consistent epithelium with in vivo-like features. In addition, molecular signatures comparable to the native tissue grade the primary 3D organoid cultures as important in vitro models for a more precise drug screening in the pharmaceutical industry in the future.

The skilled person is aware of the different types of organoids and the methods to obtain them (e.g. at least the organoids as described in the review article by Kim et al. Nature Reviews Molecular Cell Biology volume 21, pages571-584 (2020)).

If the biological material are cells, the cells can be for instance cryopreserved at a concentration of 1 x 10³ - 1x10⁹ cells/ml, preferably 1 x 10⁴ - 1x10⁸ cells/ml, more preferably 1 x 10⁵ - 1x10⁷ cells/ml, calculated on the volume of the cryopreservation formulation. If the biological material are extracellular vesicles (i.e. EV), the EVs can be for instance cryopreserved at a concentration of 1 x 10⁵ - 1x10¹¹ EVs/ml, preferably 1 x 10⁶ - 1x10¹⁰ EVs/ml, more preferably 1 x 10⁷ - 1x10⁹ EVs/ml, calculated on the volume of the cryopreservation formulation. If the biological material are organoids, the organoids can be for instance cryopreserved at a concentration of 10 - 10,000 organoids/ml, preferably 50 - 1000 organoids/ml, more preferably 100 - 500 organoids/ml, calculated on the volume of the cryopreservation formulation.

In an embodiment, the cell in the context of the current invention is a cell used in an advanced medicinal product (ATMP) and/or is one or more selected from the group consisting of a hematopoietic cell (e.g. erythroid cell, hematopoietic stem cell), myeloid cell (e.g. granulocyte, megakaryocyte, macrophage), immune cell (e.g. neutrophil, natural killer cell, T lymphocyte, B lymphocyte, monocyte, dendritic cell), peripheral blood mononuclear cell (PBMCs), stem cell (e.g. hematopoietic stem cell, induced pluripotent stem cell (iPSC), mesenchymal stem cell (MSC), embryonic stem cell), an amniotic epithelial cell, a hepatocyte, and a hepatic stellate cell.

In an embodiment, the cell in the context of the invention is a chimeric antigen receptor (CAR) T-lymphocyte. In an embodiment, the cell in the context of the invention is a CD3+ and/or CD45+ T lymphocyte. In an embodiment, the cell in the context of the invention is a CD45+and/or CD34+ stem cell.

The cryopreservation formulation of the invention is particularly suitable for the cryopreservation of one or more of "hard-to-freeze cells", "non-freezable cells", terminally-differentiated cells, and slow growing cells, for which use of 10% (v/v)(10.9 wt.%) DMSO typically does not achieve a desirable low cell death and/or high cell viability, but which typically is achieved for the combination collagen hydrolysate and DMSO.

In an embodiment, the cell in the context of the invention is an "easy-to-freeze cell". An "easy-to-freeze cell" as used herein means a cell type that shows below 20% cell death (under optimal conditions) and keeps the viability (e.g. no more than 20% reduction in viability) after cryopreservation in a formulation based on a permeating and/or non-permeating cryoprotectant as disclosed herein, other than collagen hydrolysate. Preferably, an "easy-to-freeze cell" as used herein means a cell type that shows below 20% cell death and keeps the viability (e.g. no more than 20% reduction in viability) after cryopreservation in 10% (v/v) DMSO.

In an embodiment, the cell in the context of the invention is a "hard-to-freeze cell". A "hard-to-freeze cell" as used herein means a cell type that shows below 20% cell death (under optimal conditions) but shows reduced viability (e.g. at least 20% reduction in viability) after cryopreservation in a formulation based on a permeating and/or non-permeating cryoprotectant as disclosed herein, other than collagen hydrolysate. Preferably, a "hard-to-freeze cell" as used herein means a cell type that shows below 20% cell death but shows reduced viability (e.g. at least 20% reduction in viability) after cryopreservation in 10% (v/v) DMSO.

In an embodiment, the cell in the context of the invention is a "non-freezable cell". A "non-freezable cell" as used herein means a cell type that shows at least 20% cell death (under optimal conditions) after cryopreservation in a formulation based on a permeating and/or non-permeating cryoprotectant as disclosed herein, other than collagen hydrolysate. Preferably, a "non-freezable cell" as used herein means a cell type that shows at least 20% cell death after cryopreservation in 10% (v/v) DMSO.

In an embodiment, the cell is a primary cell, meaning a cell that is (freshly) isolated from living tissue, preferably human tissue.

In an embodiment, the cell is a terminally-differentiated cell, preferably one or more selected from the group consisting of a neuronal cell, neuropil, cardiomyocyte, skeletal muscle cell, terminally-differentiated fibroblast, terminally-differentiated hepatocyte, monocyte, dendritic cell, beta-cell, pancreatic islet, human primary hepatocyte, primary human monocyte, chondrocyte, osteocyte, and adipocyte. The term "terminally differentiated cell" as used herein means a cell that hardly (i.e. no more than once per 48 h, preferably no more than once per 7 days) proliferate or does not proliferate at all, especially in vitro. The term "terminally differentiated cell" as used herein preferably means a cell that has undergone cell cycle arrest. The ability to proliferate in the context of the current invention preferably pertains to the ability to proliferate in vitro. Typically, terminally differentiated cells do not transform into other types of cells and have differentiated to perform a specific function.

In an embodiment, the biological material as disclosed herein is (intended) for transplantation into a host, preferably in a human. In an embodiment, the biological material is an advanced medicinal product (ATMP), such as one or more of a gene therapy medicinal product, a somatic cell therapy medicinal product, and tissue engineered product. For ATMP products it is typically considered as a rule of thumb, that the cell survival should be better than 80% (i.e. cell death lower than 20%), and furthermore the cryopreservation formulation should be GMP-grade and preferably DMSO and/or (animal) serum-free. The cryopreservation formulations currently available, do not meet this criteria, for instance as they often require serum (proteins) and/or DMSO. However, the cryopreservation formulation of the current invention may meet this criteria.

In an embodiment, the cryopreservation formulation is GMP (Good manufacturing practice)-grade and/or can be manufactured under GMP conditions.

In an embodiment, the cryopreservation formulation may additionally serve as a cell carrier, such as a scaffold and/or hydrogel, known in the art, including bio-inks suitable for 3D printing. The cryopreservation formulation for that purpose comprise a further crosslinkable polymer allowing formation of a hydrogel (e.g. discussed by Chaudhary et al. Beni-Suef Univ J Basic Appl Sci 11, 3 2022). For instance, the cryopreservation formulation may comprise a photo-initiator to promote crosslinking before and/or after cryopreservation, e.g. to obtain 3D constructs (Tan et al. Micromachines (Basel). 2022 Jul; 13(7): 1038).

In an embodiment, the cryopreservation formulation of the invention is suitable for long-term storage of a biological material, e.g. cells, more preferably mammalian cells, even more preferably human cells. In an embodiment, the cryopreservation formulation of the invention is used for cryopreservation for at least 1 year (e.g. 1 year - 20 years), preferably at least 5 years, more preferably at least 10 years.

If the cryopreservation formulation of the current invention is used, it surprisingly appears that the cell recovery, viability and/or functionality may be improved if no centrifugation washing step is included after thawing. In an embodiment, the biological material is not subjected to a centrifugation washing step after cryopreservation and/or after freeze-thawing, but is instead directly used for e.g. in vitro culture and/or in vivo implantation, without excluding the possibility that the cryopreservation formulation is diluted in a further (liquid) formulation of choice. The terms "after cryopreservation" and/or "after freeze-thawing" in the context of the current invention preferably mean the period of the 15 min, preferably 30 min, more preferably 60 min, even more preferably 1 day, most preferably 1 week after thawing the cells. The term "centrifugation washing" in the context of the current invention, may be any method that aggregates and/or pellets at least part of the biological material (e.g. cells) by applying a centrifugal force. Centrifugation washing allows removing the supernatant cryopreservation formulation), whereafter the biological material can be resuspended in a different aqueous medium or another carrier material. The term "centrifugation washing" encompasses the centrifugation with a commercial (lab) centrifuge, e.g. used at 50 - 10,000 x g, or 100 - 5000 x g, or 200 - 1000 x g, or 300 - 500 x g.

The terms 'comprising' or 'to comprise' and their conjugations are used in the context of the current invention in their non-limiting sense to indicate that items following the word are included, but items not specifically mentioned are not excluded.

Reference to an element by the indefinite article 'a' or 'an' does not exclude the possibility that more than one of the elements is present, unless the context clearly requires that there be one and only one of the elements. The indefinite article 'a' or 'an' thus usually means `at least one'.

In the context of the current invention, a level is "increased" or "decreased" when it is at least 1%, such as 5%, 10%, 15%, 20%, 25%, 30%, 35%, 40%, 45%, or 50% higher or lower, respectively, than the corresponding level in a control or reference. In addition or alternatively, a level in a sample may be considered increased or decreased when it is statistically significantly higher or lower, respectively, compared to a level in a control or reference

(including an earlier time point), irrespective of the size of the change. The term "to reduce" may in the context of the current invention be used interchangeably with the term "to decrease".

### FIGURE LEGENDS

Figure 1. Cell death in fibroblast 3T3 cells after cryopreservation with cryopreservation formulations based on 25% (w/v) collagen hydrolysate with 1250 EU/g LPS ("low LPS", P5000) or 13350 EU/g LPS ("high LPS", P2000) in combination with 6.7% (v/v) DMSO.
Figure 2. Cell death in fibroblast 3T3 cells in a cryopreservation formulation with 8% (v/v) DMSO, and with or without either or not 20000 EU/ml LPS ("LPS" group). Cell death was determined in absence or presence of a centrifugation washing step ("spin" group) for 3 min at 300 x g force.
Figure 3. Cell death (%) in neuropil cells after freezing in 10% (v/v) DMSO ("control") or in 6.7% (v/v) DMSO supplemented with 30% (w/v) collagen hydrolysate ("+CH").
Figure 4. Neuronal cell death after cryopreservation in various cryopreservation formulations. 30% HC = 30 w/v collagen hydrolysate with average molecular weight of ~5000 Da ("P5000").
Figure 5. Staining of glial and neuronal markers in fresh neuropil cells (left panel) and frozen neuropil cells (middle and right panel). Neuropil cells were frozen in 10% (v/v) DMSO (middle panel) or 6.7% (v/v) DMSO in combination with 30% (w/v) collagen hydrolysate (CH, "P5000"). The staining shows a combined staining of GFAP (glial marker) and MAP2 (neuronal marker).
Figure 6. Cell viability determined by staining. Fibroblast 3T3 cells were cryopreserved in freezing media without (upper panels) or with (lower panels) hydrolysed gelatin ("HG", 20 % w/v) and 0-13.3% (v/v) DMSO. Cells were thawed and cultured for 3 days. Staining for live cells was then performed.
Figure 7. Cell viability determined by count. Fibroblast 3T3 cells were cryopreserved in freezing media without (upper panels) or with (lower panels) hydrolysed gelatin ("HG", 20 % w/v) and 0-20% (v/v) DMSO. Cells were thawed and cultured for 3 days. Number of live cells were counted after 3 days.
Figure 8. Cell viability of 3D organoids as determined with the WST-1 assay 1 h post thawing (% relative to control).

### EXAMPLES

### Example 1

### Methods

Table 1 provides an overview of the starting materials used in the Examples (all from Rousselot B.V., Belgium).

**Table 1. Overview of the starting materials used. MW=molecular weight; LPS=lipopolysaccharide, EU=endotoxin units; IEP=lsoElectric Point.**

| **Material** | **Starting material** | **Weight average MW** | **LPS level** | **IEP** |
|---|---|---|---|---|
| *"P5000" hydrolysed gelatin* | Porcine skin | 4000-6000 Da | 1250 EU/g | ~8.5 |
| *"P2000" Hydrolyzed gelatin* | Porcine skin | 1000-3000 Da | 13.350 EU/g | ~8.5 |
| *"H5000" Hydrolyzed gelatin* | *Bovine Hide* | 4000-6000 Da | 3830 EU/g | ~5.5 |

The 3T3 cell line (fibroblast culture) was used in the experiments. Cells were frozen in cryovials by slow freezing using a cooling rate of - 1 °C/minute. Cells were then stored at -80 °C for at least 18 hours overnight. Freezing solutions were made by supplementing DMEM (Dulbecco's Modified Eagle Medium) culture medium with DMSO (varied between 0-13% v/v) and/or collagen hydrolysate (varied between 0-40% w/v).

For thawing, cells in the cryovial were thawed at 37°C in a water bath until only a small piece of ice was left, and the cryovial was afterwards keep on ice. The cell suspension was transferred to a tube and carefully an excess of culture medium (brought to room temperature) was added and the suspension mixed. The cells were centrifuged for 3 min at 300 x g, after which the supernatant was discarded and the cells were resuspended at the desired concentration in warm (~37 °C) cell culture medium.

The % of cell death after thawing was determined by counting the live cells and comparing it to the number of frozen cells.

### Results

Table 2. shows the cell death of fibroblast 3T3 cells after cryopreservation with cryopreservation formulations based on 0-40% (w/v) collagen hydrolysate ("P5000") and 6.7% (v/v) DMSO. It is found that a relatively high concentration of collagen hydrolysate (e.g. 30% or 40% w/v) in combination with 6.7% (v/v) DMSO reduces the cell death to below 10%. This is lower than achieved with 20% (w/v) collagen hydrolysate and 6.7% (v/v) DMSO.

**Table 2. Cell death in fibroblast 3T3 cells after cryopreservation with cryopreservation formulations based on different concentrations collagen hydrolysate (CH, "P5000") and 6.7% (v/v) DMSO.**

| **CH concentration (w/v)** | **DMSO concentration (v/v)** | **Cell death** |
|---|---|---|
| 0% | 6.7% | >20% |
| 20% | 6.7% | 2.3% |
| 30% | 6.7% | 2.9% |
| 40% | 6.7% | 6% |
| 20% | 10% | 56% |

Table 3 shows the cell death (%) in fibroblast 3T3 cells after cryopreservation with different concentrations DMSO (0%, 0.8%, 1.7%, 3.3%, 6.7% v/v), alone or in combination with 20% (w/v) collagen hydrolysate ("P5000"). It was found that 20% collagen hydrolysate can reduce cell death when used as stand-alone cryopreservative. Furthermore, it was found that cell death is reduced to below 20% for the combination of collagen hydrolysate and DMSO, even with a DMSO concentration as low as 0.8%. As compared to 10% DMSO (often regarded as the "gold standard"), the addition of collagen hydrolysate allows for a >90% reduction in the amount of DMSO concentration needed. It was also found that cell death can be reduced to 2-5% when collagen hydrolysate is used in combination with 0.8-6.7% DMSO. Such a low toxicity cannot be achieved when DMSO is used alone.

**Table 3. Cell death (%) in fibroblast 3T3 cells after cryopreservation in different concentrations DMSO (0%, 0.8%, 1.7%, 3.3%, 6.7% v/v), alone or in combination with 20% (w/v) collagen hydrolysate (CH, "P5000").**

| **DMSO concentration** | **Cell death in DMSO only** | **Cell death in DMSO + 20% CH** |
|---|---|---|
| **0%** | 75.8% | 48% |
| **0.8%** | 65.2% | 18.8% |
| **1.7%** | 48.2% | 4.1% |
| **3.3%** | 23.1% | 3.3% |
| **6.7%** | >20% | 2.3% |
| **10%** | 20% | no data |

Table 4 shows the cell death in fibroblast 3T3 cells after cryopreservation with cryopreservation formulations based on collagen hydrolysate having a molecular weight of ~2000 Da ("P2000") or ~5000 Da ("P5000") in combination with 6.7% DMSO. It is found that a collagen hydrolysate with either of molecular weight 2000 Da or 5000 Da both lead to low cell death in presence of 20%, 30% or 40% (all w/v) collagen hydrolysate.

**Table 4. Cell death in fibroblast 3T3 cells after cryopreservation with cryopreservation formulations based on collagen hydrolysate (CH) having molecular weight of ~2000 Da ("P2000" or ~5000 Da ("P5000") in combination with 6.7% DMSO.**

| **CH concentration (w/v)** | **CH ~2000 Da** | **CH ~5000 Da** |
|---|---|---|
| **0%** | 15.5% | 15.5% |
| **20%** | 8.2% | 2.3% |
| **30%** | 2.3% | 2.9% |
| **40%** | 6.0% | 6.0% |

To study the effect of a centrifugation washing step, the cells were either or not centrifuged (for 3 min at 300 x g force) and resuspended in fresh DMEM medium after freeze-thawing. The % cell death was then determined as aforementioned.

Table 5 shows the cell death in fibroblast 3T3 cells in a cryopreservation formulation with different concentrations collagen hydrolysate ("P5000") and DMSO. Cell death was determined in absence or presence of a centrifugation washing step (for 3 min at 300 x g force). It was found that the cell death is lowest in presence of 24% (w/v) collagen hydrolysate and DMSO below 8%.

**Table 5. Cell death in fibroblast 3T3 cells in a cryopreservation formulation with different concentrations collagen hydrolysate (CH, "P5000") and DMSO. Cell death was determined in absence or presence of a centrifugation washing step.**

| **Cryopreservant composition** | **Cell death without centrifugation washing** | **Cell death with centrifugation washing** |
|---|---|---|
| **0% CH + 8% DMSO** | 15.5% | 20% |
| **24% CH + 0% DMSO** | 1% | 19% |
| **24% CH + 2.7% DMSO** | 1% | 4% |
| **24% CH + 5.3% DMSO** | 2% | 4% |

Overall, it was found that the combination of 20% or more collagen hydrolysate with no or low DMSO (e.g. <10%) is most suitable for cryopreservation. Collagen hydrolysates with average molecular weights of ~2000 Da and ~5000 Da performed similar.

When testing "P5000" and "H5000" at 0 %, 10 %, 20 %, 30 %, or 40 % (w/v) in absence of DMSO, an inverse concentration-dependent relation was found between the amount of collagen hydrolysate and the % cell death. With 30% or 40% (w/v) collagen hydrolysate, the % cell death was lowest. The cells also showed a more beneficial genotype and phenotype in absence of DMSO (by DNA sequencing, methylation, cell surface marker expression, cell differentiation assays, or other functional tests).

It was found that the reduction in the amount of DMSO resulted into less changes in cell genotype and phenotype, therefore it is considered that collagen hydrolysate may reduce the negative effects of DMSO (Tunger et al. Scientific Reports volume 8, Article number: 14828, 2018).

Overall, the best results were obtained when the amount of collagen hydrolysate exceeded 20%, particularly when it was 30% or 40%.

### Example 2

### Methods

A comparison was made between "P5000" (1250 EU/g LPS) and "P2000" (13350 EU/g LPS) hydrolyzed gelatines, which differ in their LPS content. Cells were frozen and thawed according to the method in Example 1 and the % cell death was determined.

To further study the effect of LPS during washing centrifugation, freezing solutions were compared which were either or not spiked with 20000 EU/ml LPS. The freezing solution were based on DMEM according to Example 1, and containing 24% (w/v) hydrolysed gelatin *("P5000")* and 8% (v/v) DMSO. Freezing and thawing was performed according to Example 1.

After freezing and thawing, the cells were either or not centrifuged and resuspended in fresh DMEM medium. The % cell death was then determined as aforementioned.

The LPS level was determined with the Endozyme Recombinant Factor-C method. (ref: supplier Hygloss; FDA approved method).

### Results

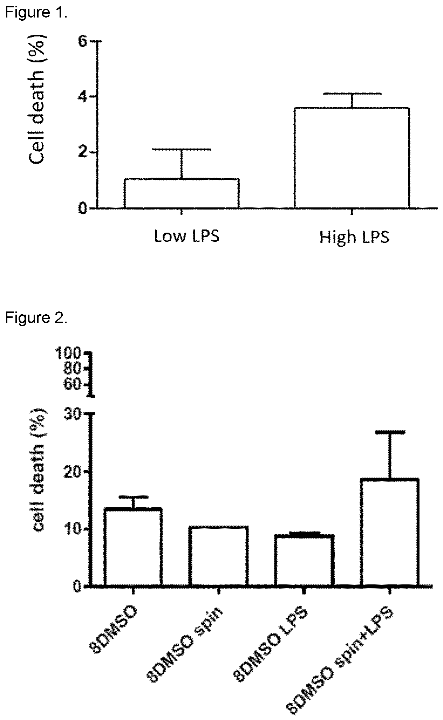

Figure 1. Cell death in fibroblast 3T3 cells after cryopreservation with cryopreservation formulations based on 25% (w/v) collagen hydrolysate with 1250 EU/g LPS ("low LPS", P5000) or 13350 EU/g LPS ("high LPS", P2000) in combination with 6.7% (v/v) DMSO. It was found that use of a collagen hydrolysate with low LPS content reduces cell death by 75% as compared to the high LPS condition.

Figure 2 shows the cell death in fibroblast 3T3 cells in a cryopreservation formulation with 8% (v/v) DMSO, and either or not spiked with 20000 EU/ml LPS ("LPS" group). Cell death was determined in absence or presence of a centrifugation washing step ("spin" group). It was found that centrifugation washing does not increase cell death in presence of 8% DMSO. However, centrifugation washing increased cell death in presence of 20000 EU/ml.

Similarly, it was found for a cryopreservation formulation comprising 24% (w/v) collagen hydrolysate ("P5000"), either or not spiked with 20000 EU/ml LPS, that a centrifugation washing step leads to higher cell death, in particular in presence of LPS. The effects of centrifugation and LPS on increased cell death were seen for cryopreservation formulations comprising 24% (w/v) collagen hydrolysate ("P5000") both with or without 8% (v/v) DMSO.

It was furthermore found that the presence of LPS during cryopreservation may induce an undesirable toxic and/or pro-inflammatory response.

Overall, it was found that cryopreservation is further improved when no or minimal LPS is present in particular when centrifugation washing is performed. Having no or low LPS may make centrifugation washing redundant.

Overall, the best results were obtained when the amount of collagen hydrolysate was around or exceeded 20% (w/v), particularly when it was 30% (w/v) or 40% (w/v).

### Example 3

### Methods

Experiments were performed with a neuropil cell population (containing neuronal cells) following the methodology as described in Example 1. The neuropil cells were used as obtained from the supplier. The neuropil cells used were around 1:3 astrocyte:neuron. Cells were stained with GFAP (glial marker) and MAP2 (neuronal marker).

### Results

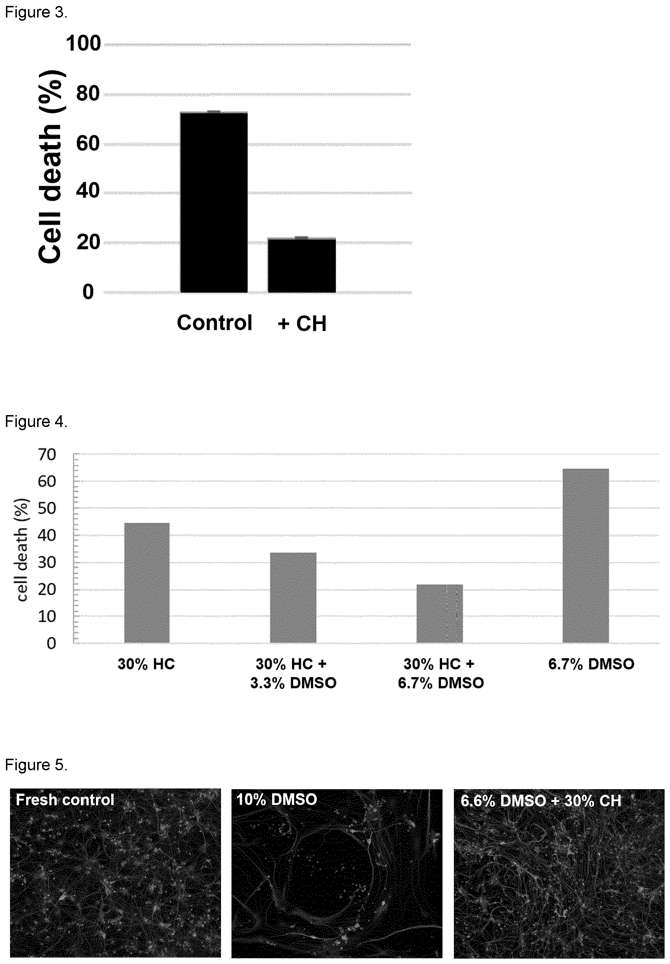

Figure 3 shows the cell death (%) in neuropil cells after freezing in 10% (v/v) DMSO ("control") or in 6.7% (v/v) DMSO supplemented with 30% (w/v) collagen hydrolysate ("+CH"). It was found that the combination of DMSO and collagen hydrolysate leads to strong reduction in cell death, which is however not seen when only 10% (v/v) DMSO is used.

Figure 4 shows the cell death in neuropil cells after cryopreservation in various cryopreservation formulations. The combination of 30% (w/v) collagen hydrolysate ("P5000") and DMSO lowers the cell death as compared to DMSO alone.

To be noted is that in the neuropil cells, cell death is almost 70% in 6.7% (v/v) DMSO, which shows the large improvement achieved by adding collagen hydrolysate.

Figure 5 shows the staining of glial and neuronal markers in fresh neuropil cells (i.e. not cryopreserved, left panel) and frozen and thawed neuropil cells (middle and right panel).

As can be seen, the left and right panels show a mix of neurons and astrocytes with dendrites at an expected density. In the middle panel, this density is lower and hence no activity is expected. When analysing the cells, the density and the formation of dendrites (the protrusion/outgrowth) were studied. It was found that neuropil cells frozen in a formulation comprising 6.7% (v/v) DMSO in combination with 30% (w/v) collagen hydrolysate ("P5000") show an improved phenotype as compared to cells frozen in a formulation of 10% (v/v) DMSO.

It is furthermore found that 20-40% (w/v) collagen hydrolysate is the optimal concentration range (with or without further DMSO).

Overall, it is found that freezing medium based on more than 20% (w/v) collagen hydrolysate is more effective than a freezing medium based on only (10% v/v) DMSO for cells that show high cell death and or low viability in 10% (v/v) DMSO (and which are therefore considered "hard-to-freeze" or "non-freezable"), such as neuronal cells. Lowest cell death and highest viability is seen if more than 20 wt.% collagen hydrolysate is combined with 5% (v/v) or other low amount of DMSO.

Similar results as for neuronal cells were obtained for non-differentiated cells, such as ESC. ESCs shows undesirable high cell death with 10% (v/v) DMSO only, however which was improved to below 5% in combination with 30% (w/v) collagen hydrolysate.

A comparison was also made between 3T3 fibroblast cells, HEK293 cells, CaLu-3 cells, mesenchymal stem cells (MSC), embryonic stem cells (ESC), and neuronal cells. It was found that 10% (v/v) or more DMSO does not lead to satisfactory cell recovery and viability in ESCs and neuronal cells. However, use of more than 20 % (w/v) collagen hydrolysate can achieve at least 80% cell recovery or better for specific cells, in particular with low concentration of DMSO (e.g. 5% or other low concentration).

Other cells that are tested include human primary cardiomyocytes, pancreatic islets, human primary hepatocytes, and primary human monocytes. For these cell types, the cell recovery in freezing in 10% (v/v) DMSO is below 20% or even below 10%. The cell recovery and viability is improved in combination with more than 20 wt.% (in particular 30 or 40 wt.%) collagen hydrolysate in combination with 1-5% (v/v) DMSO.

Overall, the best results were obtained when the amount of collagen hydrolysate exceeded 20% (v/v), particularly when it was 30% (v/v) or 40% (v/v), in combination with a low concentration (e.g. 1-5% v/v) of DMSO.

### Example 4

### Methods

Fibroblasts were cryopreserved according to example 1. After thawing, cells were cultured in tissue culture plates. The cell viability was determined after 3 days by staining for live cells and subsequently counting them.

### Results

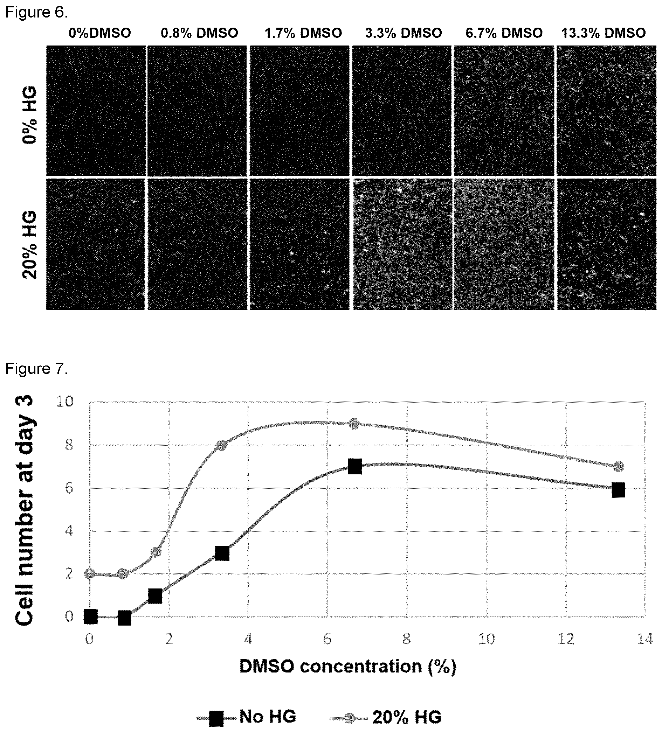

Figures 6 and 7 shows the viability of fibroblast 3T3 cells that were cryopreserved in different freezing media compositions and cultured for three days. Staining for live cells was then performed.

Over time, a relative larger cell expansion is seen for the cells that had been cryopreserved in hydrolysed gelatin in combination with DMSO, as compared to cells cryopreserved in DMSO alone. This indicates that collagen hydrolysate not only reduces the cell death, but also enhances the cell viability of cells that are recovered. It was furthermore found that cell viability was highest when the DMSO concentration was below 10% (v/v), and in particularly high for the 3.3% and 6.7% (v/v) DMSO groups. Viability was also determined after one or two days, and also showed highest cell viability when DMSO concentration was below 10% (v/v).

### Example 5

### Methods

The 3T3 cell line (fibroblast culture) was cryopreserved according to Example 1 in freezing media comprising 2.5% or 10% (v/v) DMSO, with or without 30 wt.% hydrolyzed gelatin. For the cell metabolic activity, the colorimetric MTT assay was performed at 96 h. The MTT assay was based on the metabolic reduction of MTT (3-(4,5-Dimethyl-thiazol-2-yl)-2,5-diphenyl-tetrazoliumbromide), a substance with yellow color, to a blue formazan product by mitochondrial dehydrogenases. Only viable cells were able to catalyze this reaction.

### Results

Table 6 shows the metabolic activity in fibroblast 3T3 cells after cryopreservation in the different freezing media. Highest metabolic activity is observed when cells are cryopreserved in a combination of hydrolyzed gelatin with relatively low concentration DMSO.

**Table 6. Metabolic activity in fibroblast 3T3 cells after cryopreservation in a cryopreservation formulation with different concentrations DMSO and with or without 30 wt.% hydrolyzed gelatin (collagen hydrolysate, CH).**

| **Cryopreservant composition** | **Metabolic activity** |
|---|---|
| **10% DMSO** | Average |
| **2.5% DMSO** | Low |
| **10% DMSO + 30% CH** | High |
| **2.5% DMSO + 30% CH** | Very high |

Overall it is found to be beneficial to use a relatively lower amount of DMSO. A similar effect of DMSO concentration was seen for other concentrations collagen hydrolysate.

### Example 6

### Methods

Mouse primary cortical cells (E18) were cryopreserved according to the freezing method described in Example 1, but the freezing period increased to 4 weeks. Different freezing media were compared as described in Table 7 (n=2 per group).
1. For the cell metabolic activity, the colorimetric MTT assay was performed. The MTT assay was based on the metabolic reduction of MTT (3-(4,5-Dimethyl-thiazol-2-yl)-2,5-diphenyl-tetrazoliumbromide), a substance with yellow color, to a blue formazan product by mitochondrial dehydrogenases. Only viable cells were able to catalyze this reaction.
2. Cytotoxicity was assessed in the supernatant of primary corticol neurons using the Lactate dehydrogenase (LDH) assay. LDH is a stable cytoplasmic enzyme present in all cells and rapidly released into the cell culture supernatant when the plasma membrane is damaged. The LDH activity in the cell culture supernatant was determined by a coupled enzymatic reaction in which the tetrazolium salt INT was reduced to Formazan.
3. To assess neuronal activity, primary cortical neurons were transduced with Neuroburst^{™} Lentivirus on DIV2 and the assessment of neuronal activity was monitored over 72 hours (e.g. DIV10-12) using live cell imaging of RFP oscillation for 120 sec per read on the IncuCyteTM device. The number of active cells, mean burst rate, and mean correlation were automatically evaluated using the device.

**Table 7. Different cryopreservation formulations based on DMSO and/or hydrolyzed gelatin (collagen hydrolysate, CH) tested for their effects on viability and metabolic activity in mouse primary cortical cells after cryopreservation .**

| **Cryopreservant composition** | |
|---|---|
| **DMSO (v/v)** | **CH (wt.%)** |
| **10%** | **-** |
| **-** | **30%** |
| **-** | **20%** |
| **10%** | **30%** |
| **10%** | **20%** |
| **5%** | **20%** |
| **2.5%** | **20%** |

### Results

It is found that 10% (v/v) DMSO alone leads to poor viability and metabolic activity. Overall high viability and metabolic activity is observed when DMSO is combined with collagen hydrolysate, and that the concentration of DMSO is preferably below 10% (v/v).

Similar experiments were conducted for cortical rat cells (neuropil) wherein also GFAP/MAP2 staining was performed as described in Example 3.

### Example 7

Example 7 shows the viability of intestine organoids frozen in hydrolyzed gelatins X-Pure HGP LS (Rousselot B.V., Belgium) or Peptan P5000 LD (Rousselot B.V., Belgium), compared to a freezing medium based on FCS and 10% DMSO.

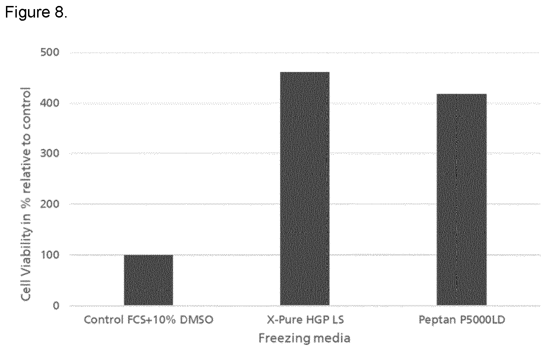

Figure 8. shows the cell viability of 3D intestinal organoids as determined with the WST-1 assay 1 h post thawing (% relative to control).

It is found that organoid viability is higher after 1 h of post thawing frozen in X-Pure HGP LS and Peptan P5000LD compared to freezing medium based on FCS + 10% DMSO.

## Claims

1. Cryopreservation formulation comprising collagen hydrolysate and dimethylsulfoxide, wherein the amount of collagen hydrolysate is 10-70 wt.% calculated on the weight of the cryopreservation formulation,
wherein the amount of dimethylsulfoxide is 1-8.5% (v/v) calculated on the volume of the cryopreservation formulation.

2. Cryopreservation formulation according to claim 1, wherein the amount of dimethylsulfoxide is 2-7% (v/v) calculated on the volume of the cryopreservation formulation.

3. Cryopreservation formulation according to claim 1 or 2, comprising 20-50 wt.% collagen hydrolysate.

4. Cryopreservation formulation according to any one of the previous claims, comprising more than 20 wt.% and less than 45 wt.% collagen hydrolysate.

5. Cryopreservation formulation according to any one of the previous claims, wherein the collagen hydrolysate is hydrolysed gelatin.

6. Cryopreservation formulation according to any one of the previous claims, wherein the collagen hydrolysate has an average molecular weight of 500-10000 Da.

7. Cryopreservation formulation according to any one of the previous claims, wherein the collagen hydrolysate has an endotoxin level of less than 10000 EU/g, preferably less than 1000 EU/g, more preferably less than 100 EU/g, calculated on the weight of the collagen hydrolysate, most preferably wherein the collagen hydrolysate is endotoxin-free.

8. Cryopreservation formulation according to any one of the previous claims, wherein the cryopreservation formulation has an endotoxin level of less than 2500 EU/ml, preferably less than 250 EU/ml, more preferably less than 25 EU/ml, calculated on the volume of the cryopreservation formulation, most preferably wherein the cryopreservation formulation is endotoxin-free.

9. Cryopreservation formulation according to any one of claims 1-8, wherein the cryopreservation formulation is free of one or more of a further cryoprotectant, serum and a serum protein.

10. Cryopreservation formulation according to any one of claims 1-8, comprising one or more of a further cryoprotectant, serum, and a serum protein.

11. Cryopreservation formulation according to claim 9 or 10, wherein the further cryoprotectant is a permeating and/or a non-permeating cryoprotectant.

12. Cryopreservation formulation according to claim 11, wherein the permeating cryoprotectant is a permeating glycol, preferably ethylene glycol, propylene glycol, or a combination thereof.

13. Cryopreservation formulation according to claim 11, wherein the non-permeating cryoprotectant is one or more selected from the group consisting of polyethylene glycol, glycerol, polyvinylpyrrolidone, methylcellulose, a sugar, or a combination thereof.

14. Method for cryopreservation of a biological material, the method comprising the step of providing the biological material in a cryopreservation formulation according to any one of claims 1-13 and reducing the temperature of cryopreservation formulation to below the freezing point of the cryopreservation formulation.

15. Method according to claim 14, wherein the biological material is selected from the group consisting of an eukaryotic cell, a prokaryotic cell, a cell organelle, an extracellular vesicle, an organoid, a tissue, and an organ.

## Patentansprüche

1. Kryokonservierungsformulierung, umfassend Kollagenhydrolysat und Dimethylsulfoxid, wobei die Menge an Kollagenhydrolysat, berechnet auf das Gewicht der Kryokonservierungsformulierung, 10-70 Gew.-% beträgt,
wobei die Menge an Dimethylsulfoxid, berechnet auf das Volumen der Kryokonservierungsformulierung, 1-8,5 Vol.-% beträgt.

2. Kryokonservierungsformulierung nach Anspruch 1, wobei die Menge an Dimethylsulfoxid, berechnet auf das Volumen der Kryokonservierungsformulierung, 2-7 Vol.-% beträgt.

3. Kryokonservierungsformulierung nach Anspruch 1 oder 2, umfassend 20-50 Gew.-% Kollagenhydrolysat.

4. Kryokonservierungsformulierung nach einem der vorherigen Ansprüche, umfassend mehr als 20 Gew.-% und weniger als 45 Gew.-% Kollagenhydrolysat.

5. Kryokonservierungsformulierung nach einem der vorherigen Ansprüche, wobei es sich bei dem Kollagenhydrolysat um hydrolysierte Gelatine handelt.

6. Kryokonservierungsformulierung nach einem der vorherigen Ansprüche, wobei das Kollagenhydrolysat ein durchschnittliches Molekulargewicht von 500-10.000 Da aufweist.

7. Kryokonservierungsformulierung nach einem der vorherigen Ansprüche, wobei das Kollagenhydrolysat einen Endotoxingehalt kleiner 10.000 EU/g, bevorzugt kleiner 1000 EU/g, bevorzugter kleiner 100 EU/g, berechnet auf das Gewicht des Kollagenhydrolysats, aufweist, wobei am meisten bevorzugt das Kollagenhydrolysat endotoxinfrei ist.

8. Kryokonservierungsformulierung nach einem der vorherigen Ansprüche, wobei die Kryokonservierungsformulierung einen Endotoxingehalt kleiner 2500 EU/ml, bevorzugt kleiner 250 EU/ml, bevorzugter kleiner 25 EU/ml, berechnet auf das Volumen der Kryokonservierungsformulierung, aufweist, wobei am meisten bevorzugt die Kryokonservierungsformulierung endotoxinfrei ist.

9. Kryokonservierungsformulierung nach einem der Ansprüche 1-8, wobei die Kryokonservierungsformulierung frei von einem oder mehreren von einem weiteren Kryoprotektivum, Serum und einem Serumprotein ist.

10. Kryokonservierungsformulierung nach einem der Ansprüche 1-8, umfassend eines oder mehrere von einem weiteren Kryoprotektivum, Serum und einem Serumprotein.

11. Kryokonservierungsformulierung nach Anspruch 9 oder 10, wobei es sich bei dem weiteren Kryoprotektivum um ein permeierendes und/oder ein nicht permeierendes Kryoprotektivum handelt.

12. Kryokonservierungsformulierung nach Anspruch 11, wobei es sich bei dem permeierenden Kryoprotektivum um ein permeierendes Glykol, bevorzugt Ethylenglykol, Propylenglykol oder eine Kombination davon, handelt.

13. Kryokonservierungsformulierung nach Anspruch 11, wobei es sich bei dem nicht permeierenden Kryoprotektivum um ein oder mehrere handelt, die aus der Gruppe bestehend aus Polyethylenglykol, Glycerin, Polyvinylpyrrolidon, Methylcellulose, einem Zucker oder einer Kombination davon ausgewählt sind.

14. Verfahren zur Kryokonservierung eines biologischen Materials, wobei das Verfahren den Schritt umfasst, bei dem das biologische Material in einer Kryokonservierungsformulierung nach einem der Ansprüche 1-13 bereitgestellt und die Temperatur der Kryokonservierungsformulierung auf unter den Gefrierpunkt der Kryokonservierungsformulierung gesenkt wird.

15. Verfahren nach Anspruch 14, wobei das biologische Material aus der Gruppe bestehend aus einer eukaryontischen Zelle, einer prokaryontischen Zelle, einer Zellorganelle, einem extrazellulären Vesikel, einem Organoid, einem Gewebe und einem Organ ausgewählt ist.

## Revendications

1. Formulation de cryoconservation comprenant un hydrolysat de collagène et du diméthylsulfoxyde, dans laquelle la quantité d'hydrolysat de collagène est de 10-70 % en poids calculée sur le poids de la formulation de cryoconservation,
dans laquelle la quantité de diméthylsulfoxyde est de 1 à 8,5 % (v/v) calculée par rapport au volume de la formulation de cryoconservation.

2. Formulation de cryoconservation selon la revendication 1, dans laquelle la quantité de diméthylsulfoxyde est de 2 à 7 % (v/v) calculée sur le volume de la formulation de cryoconservation.

3. Formulation de cryoconservation selon la revendication 1 ou 2, comprenant 20-50 % en poids d'hydrolysat de collagène.

4. Formulation de cryoconservation selon l'une quelconque des revendications précédentes, comprenant plus de 20 % en poids et moins de 45 % en poids d'hydrolysat de collagène.

5. Formulation de cryoconservation selon l'une quelconque des revendications précédentes, dans laquelle l'hydrolysat de collagène est une gélatine hydrolysée.

6. Formulation de cryoconservation selon l'une quelconque des revendications précédentes, dans laquelle l'hydrolysat de collagène a un poids moléculaire moyen de 500-10 000 Da.

7. Formulation de cryoconservation selon l'une quelconque des revendications précédentes, dans laquelle l'hydrolysat de collagène a un taux d'endotoxine inférieur à 10 000 UE/g, de préférence inférieur à 1 000 UE/g, de manière plus préférée inférieur à 100 UE/g, calculé sur le poids de l'hydrolysat de collagène, de manière particulièrement préférée dans laquelle l'hydrolysat de collagène est exempt d'endotoxine.

8. Formulation de cryoconservation selon l'une quelconque des revendications précédentes, dans laquelle la formulation de cryoconservation a un taux d'endotoxine inférieur à 2 500 UE/ml, de préférence inférieur à 250 UE/ml, de manière plus préférée inférieur à 25 UE/ml, calculé sur le volume de la formulation de cryoconservation, de manière particulièrement préférée dans laquelle la formulation de cryoconservation est exempte d'endotoxine.

9. Formulation de cryoconservation selon l'une quelconque des revendications 1 à 8, dans laquelle la formulation de cryoconservation est exempte d'un ou plusieurs parmi un cryoprotecteur supplémentaire, du sérum et une protéine sérique.

10. Formulation de cryoconservation selon l'une quelconque des revendications 1 à 8, comprenant un ou plusieurs parmi un cryoprotecteur supplémentaire, du sérum et une protéine sérique.

11. Formulation de cryoconservation selon la revendication 9 ou 10, dans laquelle le cryoprotecteur supplémentaire est un cryoprotecteur perméant et/ou non perméant.

12. Formulation de cryoconservation selon la revendication 11, dans laquelle le cryoprotecteur perméant est un glycol perméant, de préférence l'éthylèneglycol, le propylèneglycol, ou une combinaison de ceux-ci.

13. Formulation de cryoconservation selon la revendication 11, dans laquelle le cryoprotecteur non perméant est un ou plusieurs choisis dans le groupe constitué par le polyéthylène glycol, le glycérol, la polyvinylpyrrolidone, la méthylcellulose, un sucre, ou une combinaison de ceux-ci.

14. Procédé de cryoconservation d'une matière biologique, le procédé comprenant l'étape de fourniture de la matière biologique dans une formulation de cryoconservation selon l'une quelconque des revendications 1-13 et réduction de la température de la formulation de cryoconservation jusqu'à une température inférieure au point de congélation de la formulation de cryoconservation.

15. Procédé selon la revendication 14, dans lequel la matière biologique est choisie dans le groupe constitué par une cellule eucaryote, une cellule procaryote, un organite cellulaire, une vésicule extracellulaire, un organoïde, un tissu et un organe.
